# EUROPÄISCHE PATENTANMELDUNG

(11) **EP 1 894 999 A1**
(43) Veröffentlichungstag der Anmeldung: **05.03.2008**
(21) Anmeldenummer: 06120008.5
(22) Anmeldetag: 01.09.2006
(51) Int. Cl.: C12N 9/02, C07C 5/09, C12P 7/00

(54) **Verfahren zur enzymatischen Reduktion von Alkinderivaten**

(71) Anmelder: BASF SE, 67056 Ludwigshafen (DE)
(72) Erfinder: Stürmer, Rainer, Dr., 67127 Rödersheim-Gronau (DE); Hauer, Bernhard, Prof. Dr., 67136 Fußgönheim (DE); Rosche, Bettina, Dr., Randwick NSW 2031 (AU); Müller, André, Sydney NSW (AU)
(74) Vertreter: Reitstötter - Kinzebach

(57) **Zusammenfassung**

Die Erfindung betrifft ein Verfahren zur enzymatischen Reduktion von Alkinderivaten der Formel (1) 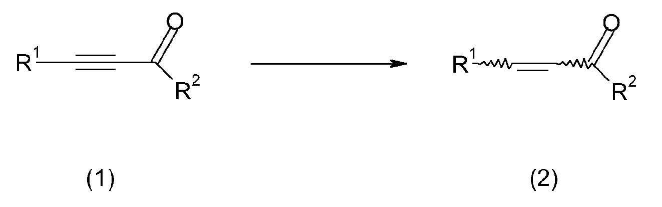 worin
R¹ für H, C₁-C₆- Alkyl, C₂-C₆- Alkenyl, oder einen gegebenenfalls substituierten carbo- oder heterocyclischen, aromatischen oder nichtaromatischen Rest steht,
R² für H, C₁-C₆- Alkyl oder C₂-C₆-Alkenyl steht
durch Umsetzung in Gegenwart einer spezieller Reduktasen.

SEQUENCE LISTING

<110>  BASF Aktiengesellschaft

<120>  Verfahren zur enzymatischen Reduktion von Alkinderivaten

<130>  M/47256

<160>  9

<170>  PatentIn version 3.3

<210>  1
<211>  399
<212>  PRT
<213>  Saccharomyces carlsbergensis

<400>  1

Ser Phe Val Lys Asp Phe Lys Pro Gln Ala Leu Gly Asp Thr Asn Leu
1               5                   10                  15

Phe Lys Pro Ile Lys Ile Gly Asn Asn Glu Leu Leu His Arg Ala Val
            20                  25                  30

Ile Pro Pro Leu Thr Arg Met Arg Ala Leu His Pro Gly Asn Ile Pro
        35                  40                  45

Asn Arg Asp Trp Ala Val Glu Tyr Tyr Thr Gln Arg Ala Gln Arg Pro
    50                  55                  60

Gly Thr Met Ile Ile Thr Glu Gly Ala Phe Ile Ser Pro Gln Ala Gly
65                  70                  75                  80

Gly Tyr Asp Asn Ala Pro Gly Val Trp Ser Glu Glu Gln Met Val Glu
                85                  90                  95

Trp Thr Lys Ile Phe Asn Ala Ile His Glu Lys Lys Ser Phe Val Trp
            100                 105                 110

Val Gln Leu Trp Val Leu Gly Trp Ala Ala Phe Pro Asp Asn Leu Ala
        115                 120                 125

Arg Asp Gly Leu Arg Tyr Asp Ser Ala Ser Asp Asn Val Phe Met Asp
    130                 135                 140

Ala Glu Gln Glu Ala Lys Ala Lys Lys Ala Asn Asn Pro Gln His Ser
145                 150                 155                 160

Leu Thr Lys Asp Glu Ile Lys Gln Tyr Ile Lys Glu Tyr Val Gln Ala
                165                 170                 175

Ala Lys Asn Ser Ile Ala Ala Gly Ala Asp Gly Val Glu Ile His Ser
            180                 185                 190

Ala Asn Gly Tyr Leu Leu Asn Gln Phe Leu Asp Pro His Ser Asn Thr
        195                 200                 205

Arg Thr Asp Glu Tyr Gly Gly Ser Ile Glu Asn Arg Ala Arg Phe Thr
    210                 215                 220

Leu Glu Val Val Asp Ala Leu Val Glu Ala Ile Gly His Glu Lys Val
225                 230                 235                 240

Gly Leu Arg Leu Ser Pro Tyr Gly Val Phe Asn Ser Met Ser Gly Gly
                245                 250                 255

Ala Glu Thr Gly Ile Val Ala Gln Tyr Ala Tyr Val Ala Gly Glu Leu
            260                 265                 270

Glu Lys Arg Ala Lys Ala Gly Lys Arg Leu Ala Phe Val His Leu Val
        275                 280                 285

Glu Pro Arg Val Thr Asn Pro Phe Leu Thr Glu Gly Glu Gly Glu Tyr
    290                 295                 300

Glu Gly Gly Ser Asn Asp Phe Val Tyr Ser Ile Trp Lys Gly Pro Val
305                 310                 315                 320

Ile Arg Ala Gly Asn Phe Ala Leu His Pro Glu Val Val Arg Glu Glu
                325                 330                 335

Val Lys Asp Lys Arg Thr Leu Ile Gly Tyr Gly Arg Phe Phe Ile Ser
            340                 345                 350

Asn Pro Asp Leu Val Asp Arg Leu Glu Lys Gly Leu Pro Leu Asn Lys
        355                 360                 365

Tyr Asp Arg Asp Thr Phe Tyr Gln Met Ser Ala His Gly Tyr Ile Asp
    370                 375                 380

Tyr Pro Thr Tyr Glu Glu Ala Leu Lys Leu Gly Trp Asp Lys Lys
385                 390                 395

<210>  2
<211>  399
<212>  PRT
<213>  Saccharomyces cerevisiae

<400>  2

Pro Phe Val Lys Asp Phe Lys Pro Gln Ala Leu Gly Asp Thr Asn Leu
1               5                   10                  15

Phe Lys Pro Ile Lys Ile Gly Asn Asn Glu Leu Leu His Arg Ala Val
            20                  25                  30

Ile Pro Pro Leu Thr Arg Met Arg Ala Gln His Pro Gly Asn Ile Pro
        35                  40                  45

Asn Arg Asp Trp Ala Val Glu Tyr Tyr Ala Gln Arg Ala Gln Arg Pro
    50                  55                  60

Gly Thr Leu Ile Ile Thr Glu Gly Thr Phe Pro Ser Pro Gln Ser Gly
65                  70                  75                  80

Gly Tyr Asp Asn Ala Pro Gly Ile Trp Ser Glu Glu Gln Ile Lys Glu
                85                  90                  95

Trp Thr Lys Ile Phe Lys Ala Ile His Glu Asn Lys Ser Phe Ala Trp
            100                 105                 110

Val Gln Leu Trp Val Leu Gly Trp Ala Ala Phe Pro Asp Thr Leu Ala
        115                 120                 125

Arg Asp Gly Leu Arg Tyr Asp Ser Ala Ser Asp Asn Val Tyr Met Asn
    130                 135                 140

Ala Glu Gln Glu Glu Lys Ala Lys Lys Ala Asn Asn Pro Gln His Ser
145                 150                 155                 160

Ile Thr Lys Asp Glu Ile Lys Gln Tyr Val Lys Glu Tyr Val Gln Ala
                165                 170                 175

Ala Lys Asn Ser Ile Ala Ala Gly Ala Asp Gly Val Glu Ile His Ser
            180                 185                 190

Ala Asn Gly Tyr Leu Leu Asn Gln Phe Leu Asp Pro His Ser Asn Asn
        195                 200                 205

Arg Thr Asp Glu Tyr Gly Gly Ser Ile Glu Asn Arg Ala Arg Phe Thr
    210                 215                 220

Leu Glu Val Val Asp Ala Val Val Asp Ala Ile Gly Pro Glu Lys Val
225                 230                 235                 240

Gly Leu Arg Leu Ser Pro Tyr Gly Val Phe Asn Ser Met Ser Gly Gly
                245                 250                 255

Ala Glu Thr Gly Ile Val Ala Gln Tyr Ala Tyr Val Leu Gly Glu Leu
            260                 265                 270

Glu Arg Arg Ala Lys Ala Gly Lys Arg Leu Ala Phe Val His Leu Val
        275                 280                 285

Glu Pro Arg Val Thr Asn Pro Phe Leu Thr Glu Gly Glu Gly Glu Tyr
    290                 295                 300

Asn Gly Gly Ser Asn Lys Phe Ala Tyr Ser Ile Trp Lys Gly Pro Ile
305                 310                 315                 320

Ile Arg Ala Gly Asn Phe Ala Leu His Pro Glu Val Val Arg Glu Glu
                325                 330                 335

Val Lys Asp Pro Arg Thr Leu Ile Gly Tyr Gly Arg Phe Phe Ile Ser
            340                 345                 350

Asn Pro Asp Leu Val Asp Arg Leu Glu Lys Gly Leu Pro Leu Asn Lys
        355                 360                 365

Tyr Asp Arg Asp Thr Phe Tyr Lys Met Ser Ala Glu Gly Tyr Ile Asp
    370                 375                 380

Tyr Pro Thr Tyr Glu Glu Ala Leu Lys Leu Gly Trp Asp Lys Asn
385                 390                 395

<210>  3
<211>  399
<212>  PRT
<213>  Saccharomyces cerevisiae

<400>  3

Pro Phe Val Lys Gly Phe Glu Pro Ile Ser Leu Arg Asp Thr Asn Leu
1               5                   10                  15

Phe Glu Pro Ile Lys Ile Gly Asn Thr Gln Leu Ala His Arg Ala Val
            20                  25                  30

Met Pro Pro Leu Thr Arg Met Arg Ala Thr His Pro Gly Asn Ile Pro
        35                  40                  45

Asn Lys Glu Trp Ala Ala Val Tyr Tyr Gly Gln Arg Ala Gln Arg Pro
    50                  55                  60

Gly Thr Met Ile Ile Thr Glu Gly Thr Phe Ile Ser Pro Gln Ala Gly
65                  70                  75                  80

Gly Tyr Asp Asn Ala Pro Gly Ile Trp Ser Asp Glu Gln Val Ala Glu
                85                  90                  95

Trp Lys Asn Ile Phe Leu Ala Ile His Asp Cys Gln Ser Phe Ala Trp
            100                 105                 110

Val Gln Leu Trp Ser Leu Gly Trp Ala Ser Phe Pro Asp Val Leu Ala
        115                 120                 125

Arg Asp Gly Leu Arg Tyr Asp Cys Ala Ser Asp Arg Val Tyr Met Asn
    130                 135                 140

Ala Thr Leu Gln Glu Lys Ala Lys Asp Ala Asn Asn Leu Glu His Ser
145                 150                 155                 160

Leu Thr Lys Asp Asp Ile Lys Gln Tyr Ile Lys Asp Tyr Ile His Ala
                165                 170                 175

Ala Lys Asn Ser Ile Ala Ala Gly Ala Asp Gly Val Glu Ile His Ser
            180                 185                 190

Ala Asn Gly Tyr Leu Leu Asn Gln Phe Leu Asp Pro His Ser Asn Lys
        195                 200                 205

Arg Thr Asp Glu Tyr Gly Gly Thr Ile Glu Asn Arg Ala Arg Phe Thr
    210                 215                 220

Leu Glu Val Val Asp Ala Leu Ile Glu Thr Ile Gly Pro Glu Arg Val
225                 230                 235                 240

Gly Leu Arg Leu Ser Pro Tyr Gly Thr Phe Asn Ser Met Ser Gly Gly
                245                 250                 255

Ala Glu Pro Gly Ile Ile Ala Gln Tyr Ser Tyr Val Leu Gly Glu Leu
            260                 265                 270

Glu Lys Arg Ala Lys Ala Gly Lys Arg Leu Ala Phe Val His Leu Val
        275                 280                 285

Glu Pro Arg Val Thr Asp Pro Ser Leu Val Glu Gly Glu Gly Glu Tyr
    290                 295                 300

Ser Glu Gly Thr Asn Asp Phe Ala Tyr Ser Ile Trp Lys Gly Pro Ile
305                 310                 315                 320

Ile Arg Ala Gly Asn Tyr Ala Leu His Pro Glu Val Val Arg Glu Gln
                325                 330                 335

Val Lys Asp Pro Arg Thr Leu Ile Gly Tyr Gly Arg Phe Phe Ile Ser
            340                 345                 350

Asn Pro Asp Leu Val Tyr Arg Leu Glu Glu Gly Leu Pro Leu Asn Lys
        355                 360                 365

Tyr Asp Arg Ser Thr Phe Tyr Thr Met Ser Ala Glu Gly Tyr Thr Asp
    370                 375                 380

Tyr Pro Thr Tyr Glu Glu Ala Val Asp Leu Gly Trp Asn Lys Asn
385                 390                 395

<210>  4
<211>  5490
<212>  DNA
<213>  Artificial

<220>
<223>  Plamid encoding OYE1

<220>
<221>  CDS
<222>  (4)..(1203)

<400>  4
cat atg agc ttt gtg aaa gat ttt aaa ccg cag gcg ctg ggc gat acc      48
    Met Ser Phe Val Lys Asp Phe Lys Pro Gln Ala Leu Gly Asp Thr
    1               5                   10                  15

aac ctg ttt aaa ccg att aaa att ggc aac aac gaa ctg ctg cat cgc      96
Asn Leu Phe Lys Pro Ile Lys Ile Gly Asn Asn Glu Leu Leu His Arg
                20                  25                  30

gcg gtg att ccg ccg ctg acc cgc atg cgc gcg ctg cat ccg ggc aac      144
Ala Val Ile Pro Pro Leu Thr Arg Met Arg Ala Leu His Pro Gly Asn
            35                  40                  45

att ccg aac cgc gat tgg gcg gtg gaa tat tat acc cag cgc gcg cag      192
Ile Pro Asn Arg Asp Trp Ala Val Glu Tyr Tyr Thr Gln Arg Ala Gln
        50                  55                  60

cgc ccg ggc acc atg att att acc gaa ggc gcg ttt att agc ccg cag      240
Arg Pro Gly Thr Met Ile Ile Thr Glu Gly Ala Phe Ile Ser Pro Gln
    65                  70                  75

gcg ggc ggc tat gat aac gcg ccg ggc gtg tgg agc gaa gaa cag atg      288
Ala Gly Gly Tyr Asp Asn Ala Pro Gly Val Trp Ser Glu Glu Gln Met
80                  85                  90                  95

gtg gaa tgg acc aaa att ttt aac gcg att cat gaa aaa aaa agc ttt      336
Val Glu Trp Thr Lys Ile Phe Asn Ala Ile His Glu Lys Lys Ser Phe
                100                 105                 110

gtg tgg gtg cag ctg tgg gtg ctg ggc tgg gcg gcg ttt ccg gat aac      384
Val Trp Val Gln Leu Trp Val Leu Gly Trp Ala Ala Phe Pro Asp Asn
            115                 120                 125

ctg gcg cgc gat ggc ctg cgc tat gat agc gcg agc gat aac gtg ttt      432
Leu Ala Arg Asp Gly Leu Arg Tyr Asp Ser Ala Ser Asp Asn Val Phe
        130                 135                 140

atg gat gcg gaa cag gaa gcg aaa gcg aaa aaa gcg aac aac ccg cag      480
Met Asp Ala Glu Gln Glu Ala Lys Ala Lys Lys Ala Asn Asn Pro Gln
    145                 150                 155

cat agc ctg acc aaa gat gaa att aaa cag tat att aaa gaa tat gtg      528
His Ser Leu Thr Lys Asp Glu Ile Lys Gln Tyr Ile Lys Glu Tyr Val
160                 165                 170                 175

cag gcg gcg aaa aac agc att gcg gcg ggc gcg gat ggc gtg gaa att      576
Gln Ala Ala Lys Asn Ser Ile Ala Ala Gly Ala Asp Gly Val Glu Ile
                180                 185                 190

cat agc gcg aac ggc tat ctg ctg aac cag ttt ctg gat ccg cat agc      624
His Ser Ala Asn Gly Tyr Leu Leu Asn Gln Phe Leu Asp Pro His Ser
            195                 200                 205

aac acc cgc acc gat gaa tat ggc ggc agc att gaa aac cgc gcg cgc      672
Asn Thr Arg Thr Asp Glu Tyr Gly Gly Ser Ile Glu Asn Arg Ala Arg
        210                 215                 220

ttt acc ctg gaa gtg gtg gat gcg ctg gtg gaa gcg att ggc cat gaa      720
Phe Thr Leu Glu Val Val Asp Ala Leu Val Glu Ala Ile Gly His Glu

225                 230                 235

aaa gtg ggc ctg cgc ctg agc ccg tat ggc gtg ttt aac agc atg agc      768
Lys Val Gly Leu Arg Leu Ser Pro Tyr Gly Val Phe Asn Ser Met Ser
240                 245                 250                 255

ggc ggc gcg gaa acc ggc att gtg gcg cag tat gcg tat gtg gcg ggc      816
Gly Gly Ala Glu Thr Gly Ile Val Ala Gln Tyr Ala Tyr Val Ala Gly
                260                 265                 270

gaa ctg gaa aaa cgc gcg aaa gcg ggc aaa cgc ctg gcg ttt gtg cat      864
Glu Leu Glu Lys Arg Ala Lys Ala Gly Lys Arg Leu Ala Phe Val His
            275                 280                 285

ctg gtg gaa ccg cgc gtg acc aac ccg ttt ctg acc gaa ggc gaa ggc      912
Leu Val Glu Pro Arg Val Thr Asn Pro Phe Leu Thr Glu Gly Glu Gly
        290                 295                 300

gaa tat gaa ggc ggc agc aac gat ttt gtg tat agc att tgg aaa ggc      960
Glu Tyr Glu Gly Gly Ser Asn Asp Phe Val Tyr Ser Ile Trp Lys Gly
    305                 310                 315

ccg gtg att cgc gcg ggc aac ttt gcg ctg cat ccg gaa gtg gtg cgc     1008
Pro Val Ile Arg Ala Gly Asn Phe Ala Leu His Pro Glu Val Val Arg
320                 325                 330                 335

gaa gaa gtg aaa gat aaa cgc acc ctg att ggc tat ggc cgc ttt ttt     1056
Glu Glu Val Lys Asp Lys Arg Thr Leu Ile Gly Tyr Gly Arg Phe Phe
                340                 345                 350

att agc aac ccg gat ctg gtg gat cgc ctg gaa aaa ggc ctg ccg ctg     1104
Ile Ser Asn Pro Asp Leu Val Asp Arg Leu Glu Lys Gly Leu Pro Leu
            355                 360                 365

aac aaa tat gat cgc gat acc ttt tat cag atg agc gcg cat ggc tat     1152
Asn Lys Tyr Asp Arg Asp Thr Phe Tyr Gln Met Ser Ala His Gly Tyr
        370                 375                 380

att gat tat ccg acc tat gaa gaa gcg ctg aaa ctg ggc tgg gat aaa     1200
Ile Asp Tyr Pro Thr Tyr Glu Glu Ala Leu Lys Leu Gly Trp Asp Lys
    385                 390                 395

aaa tgacttaact gcagccaagc ttggctgttt tggcggatga gagaagattt          1253
Lys
400

tcagcctgat acagattaaa tcagaacgca gaagcggtct gataaaacag aatttgcctg   1313

gcggcagtag cgcggtggtc ccacctgacc ccatgccgaa ctcagaagtg aaacgccgta   1373

gcgccgatgg tagtgtgggg tctccccatg cgagagtagg gaactgccag gcatcaaata   1433

aaacgaaagg ctcagtcgaa agactgggcc tttcgtttta tctgttgttt gtcggtgaac   1493

gctctcctga gtaggacaaa tccgccggga gcggatttga acgttgcgaa gcaacggccc   1553

ggagggtggc gggcaggacg cccgccataa actgccaggc atcaaattaa gcagaaggcc   1613

atcctgacgg atggcctttt tgcgtttcta caaactcttt tgtttatttt tctaaataca   1673

ttcaaatatg tatccgctca tgagacaata accctgataa atgcttcaat aatattgaaa   1733

aggaagagta tgagtattca acatttccgt gtcgccctta ttcccttttt tgcggcattt   1793

tgccttcctg tttttgctca cccagaaacg ctggtgaaag taaaagatgc tgaagatcag   1853

ttgggtgcac gagtgggtta catcgaactg gatctcaaca gcggtaagat ccttgagagt   1913

tttcgccccg aagaacgttt tccaatgatg agcactttta aagttctgct atgtggcgcg   1973

gtattatccc gtgttgacgc cgggcaagag caactcggtc gccgcataca ctattctcag   2033

aatgacttgg ttgagtactc accagtcaca gaaaagcatc ttacggatgg catgacagta   2093

agagaattat gcagtgctgc cataaccatg agtgataaca ctgcggccaa cttacttctg   2153

acaacgatcg gaggaccgaa ggagctaacc gcttttttgc acaacatggg ggatcatgta   2213

actcgccttg atcgttggga accggagctg aatgaagcca taccaaacga cgagcgtgac   2273

accacgatgc ctgtagcaat ggcaacaacg ttgcgcaaac tattaactgg cgaactactt   2333

actctagctt cccggcaaca attaatagac tggatggagg cggataaagt tgcaggacca 2393
cttctgcgct cggcccttcc ggctggctgg tttattgctg ataaatctgg agccggtgag 2453
cgtgggtctc gcggtatcat tgcagcactg gggccagatg gtaagccctc ccgtatcgta 2513
gttatctaca cgacggggag tcaggcaact atggatgaac gaaatagaca gatcgctgag 2573
ataggtgcct cactgattaa gcattggtaa ctgtcagacc aagtttactc atatatactt 2633
tagattgatt taaaacttca tttttaattt aaaaggatct aggtgaagat cctttttgat 2693
aatctcatga ccaaaatccc ttaacgtgag ttttcgttcc actgagcgtc agaccccgta 2753
gaaaagatca aaggatcttc ttgagatcct ttttttctgc gcgtaatctg ctgcttgcaa 2813
acaaaaaaac caccgctacc agcggtggtt tgtttgccgg atcaagagct accaactctt 2873
tttccgaagg taactggctt cagcagagcg cagataccaa atactgtcct tctagtgtag 2933
ccgtagttag gccaccactt caagaactct gtagcaccgc ctacatacct cgctctgcta 2993
atcctgttac cagtggctgc tgccagtggc gataagtcgt gtcttaccgg gttggactca 3053
agacgatagt taccggataa ggcgcagcgg tcgggctgaa cggggggttc gtgcacacag 3113
cccagcttgg agcgaacgac ctacaccgaa ctgagatacc tacagcgtga gctatgagaa 3173
agcgccacgc ttcccgaagg gagaaaggcg gacaggtatc cggtaagcgg cagggtcgga 3233
acaggagagc gcacgaggga gcttccaggg ggaaacgcct ggtatcttta tagtcctgtc 3293
gggtttcgcc acctctgact tgagcgtcga tttttgtgat gctcgtcagg ggggcggagc 3353
ctatggaaaa acgccagcaa cgcggccttt ttacggttcc tggccttttg ctggcctttt 3413
gctcacatgt tctttcctgc gttatcccct gattctgtgg ataaccgtat taccgccttt 3473
gagtgagctg ataccgctcg ccgcagccga acgaccgagc gcagcgagtc agtgagcgag 3533
gaagcggaag agcgcctgat gcggtatttt ctccttacgc atctgtgcgg tatttcacac 3593
cgcatatatg gtgcactctc agtacaatct gctctgatgc cgcatagtta agccagtata 3653
cactccgcta tcgctacgtg actgggtcat ggctgcgccc cgacacccgc caacacccgc 3713
tgacgcgccc tgacgggctt gtctgctccc ggcatccgct tacagacaag ctgtgaccgt 3773
ctccgggagc tgcatgtgtc agaggttttc accgtcatca ccgaaacgcg cgaggcagct 3833
gcggtaaagc tcatcagcgt ggtcgtgaag cgattcacag atgtctgcct gttcatccgc 3893
gtccagctcg ttgagtttct ccagaagcgt taatgtctgg cttctgataa agcgggccat 3953
gttaagggcg gttttttcct gtttggtcac ttgatgcctc cgtgtaaggg ggaatttctg 4013
ttcatggggg taatgatacc gatgaaacga gagaggatgc tcacgatacg ggttactgat 4073
gatgaacatg cccggttact ggaacgttgt gagggtaaac aactggcggt atggatgcgg 4133
cgggaccaga gaaaaatcac tcagggtcaa tgccagcgct tcgttaatac agatgtaggt 4193
gttccacagg gtagccagca gcatcctgcg atgcagatcc ggaacataat ggtgcagggc 4253
gctgacttcc gcgtttccag actttacgaa acacggaaac cgaagaccat tcatgttgtt 4313
gctcaggtcg cagacgtttt gcagcagcag tcgcttcacg ttcgctcgcg tatcggtgat 4373
tcattctgct aaccagtaag gcaaccccgc cagcctagcc gggtcctcaa cgacaggagc 4433
acgatcatgc gcacccgtgg ccaggaccca acgctgcccg agatgcgccg cgtgcggctg 4493
ctggagatgg cggacgcgat ggatatgttc tgccaagggt tggtttgcgc attcacagtt 4553
ctccgcaaga attgattggc tccaattctt ggagtggtga atccgttagc gaggtgccgc 4613
cggcttccat tcaggtcgag gtggcccggc tccatgcacc gcgacgcaac gcggggaggc 4673
agacaaggta taggcggcgc cctacaatcc atgccaaccc gttccatgtg ctcgccgagg 4733

cggcataaat cgccgtgacg atcagcggtc cagtgatcga agttaggctg gtaagagccg 4793

cgagcgatcc ttgaagctgt ccctgatggt cgtcatctac ctgcctggac agcatggcct 4853

gcaacgcggg catcccgatg ccgccggaag cgagaagaat cataatgggg aaggccatcc 4913

agcctcgcgt cgcgaacgcc agcaagacgt agcccagcgc gtcggccgcc atgccggcga 4973

taatggcctg cttctcgccg aaacgtttgg tggcgggacc agtgacgaag gcttgagcga 5033

gggcgtgcaa gattccgaat accgcaagcg acaggccgat catcgtcgcg ctccagcgaa 5093

agcggtcctc gccgaaaatg acccagagcg ctgccggcac ctgtcctacg agttgcatga 5153

taaagaagac agtcataagt gcggcgacga tagtcatgcc ccgcgcccac cggaaggagc 5213

tgactgggtt gaaggctctc aagggcatcg gtcgacgctc tcccttatgc gactcctgca 5273

ttaggaagca gcccagtagt aggttgaggc cgttgagcac cgccgccgca aggaatggtg 5333

catgcatcga tcaccacaat tcagcaaatt gtgaacatca tcacgttcat ctttccctgg 5393

ttgccaatgg cccattttcc tgtcagtaac gagaaggtcg cgaattcagg cgctttttag 5453

actggtcgta atgaacaatt cttaagaagg agatata 5490

<210> 5
<211> 400
<212> PRT
<213> Artificial

<220>
<223> Synthetic Construct

<400> 5

Met Ser Phe Val Lys Asp Phe Lys Pro Gln Ala Leu Gly Asp Thr Asn
1               5                   10                  15

Leu Phe Lys Pro Ile Lys Ile Gly Asn Asn Glu Leu Leu His Arg Ala
            20                  25                  30

Val Ile Pro Pro Leu Thr Arg Met Arg Ala Leu His Pro Gly Asn Ile
        35                  40                  45

Pro Asn Arg Asp Trp Ala Val Glu Tyr Tyr Thr Gln Arg Ala Gln Arg
    50                  55                  60

Pro Gly Thr Met Ile Ile Thr Glu Gly Ala Phe Ile Ser Pro Gln Ala
65                  70                  75                  80

Gly Gly Tyr Asp Asn Ala Pro Gly Val Trp Ser Glu Glu Gln Met Val
                85                  90                  95

Glu Trp Thr Lys Ile Phe Asn Ala Ile His Glu Lys Lys Ser Phe Val
            100                 105                 110

Trp Val Gln Leu Trp Val Leu Gly Trp Ala Ala Phe Pro Asp Asn Leu
        115                 120                 125

Ala Arg Asp Gly Leu Arg Tyr Asp Ser Ala Ser Asp Asn Val Phe Met
    130                 135                 140

Asp Ala Glu Gln Glu Ala Lys Ala Lys Lys Ala Asn Asn Pro Gln His
145                 150                 155                 160

Ser Leu Thr Lys Asp Glu Ile Lys Gln Tyr Ile Lys Glu Tyr Val Gln
                165                 170                 175

Ala Ala Lys Asn Ser Ile Ala Ala Gly Ala Asp Gly Val Glu Ile His
            180                 185                 190

Ser Ala Asn Gly Tyr Leu Leu Asn Gln Phe Leu Asp Pro His Ser Asn
        195                 200                 205

Thr Arg Thr Asp Glu Tyr Gly Gly Ser Ile Glu Asn Arg Ala Arg Phe
    210                 215                 220

Thr Leu Glu Val Val Asp Ala Leu Val Glu Ala Ile Gly His Glu Lys
225                 230                 235                 240

Val Gly Leu Arg Leu Ser Pro Tyr Gly Val Phe Asn Ser Met Ser Gly
                245                 250                 255

Gly Ala Glu Thr Gly Ile Val Ala Gln Tyr Ala Tyr Val Ala Gly Glu
            260                 265                 270

Leu Glu Lys Arg Ala Lys Ala Gly Lys Arg Leu Ala Phe Val His Leu
        275                 280                 285

Val Glu Pro Arg Val Thr Asn Pro Phe Leu Thr Glu Gly Glu Gly Glu
    290                 295                 300

Tyr Glu Gly Gly Ser Asn Asp Phe Val Tyr Ser Ile Trp Lys Gly Pro
305                 310                 315                 320

Val Ile Arg Ala Gly Asn Phe Ala Leu His Pro Glu Val Val Arg Glu
                325                 330                 335

Glu Val Lys Asp Lys Arg Thr Leu Ile Gly Tyr Gly Arg Phe Phe Ile
            340                 345                 350

Ser Asn Pro Asp Leu Val Asp Arg Leu Glu Lys Gly Leu Pro Leu Asn
        355                 360                 365

Lys Tyr Asp Arg Asp Thr Phe Tyr Gln Met Ser Ala His Gly Tyr Ile
    370                 375                 380

Asp Tyr Pro Thr Tyr Glu Glu Ala Leu Lys Leu Gly Trp Asp Lys Lys
385                 390                 395                 400

<210>  6
<211>  5491
<212>  DNA
<213>  Artificial

<220>
<223>  Plasmid encoding OYE2

<220>
<221>  CDS
<222>  (4)..(1203)

<400>  6
cat atg ccg ttt gtg aaa gat ttt aaa ccg cag gcg ctg ggc gat acc        48
    Met Pro Phe Val Lys Asp Phe Lys Pro Gln Ala Leu Gly Asp Thr
    1               5                   10                  15

aac ctg ttt aaa ccg att aaa att ggc aac aac gaa ctg ctg cat cgc        96
Asn Leu Phe Lys Pro Ile Lys Ile Gly Asn Asn Glu Leu Leu His Arg
                20                  25                  30

gcg gtg att ccg ccg ctg acc cgc atg cgc gcg cag cat ccg ggc aac       144
Ala Val Ile Pro Pro Leu Thr Arg Met Arg Ala Gln His Pro Gly Asn
            35                  40                  45

att ccg aac cgc gat tgg gcg gtg gaa tat tat gcg cag cgc gcg cag 192
Ile Pro Asn Arg Asp Trp Ala Val Glu Tyr Tyr Ala Gln Arg Ala Gln
        50                  55                  60

cgc ccg ggc acc ctg att att acc gaa ggc acc ttt ccg agc ccg cag 240
Arg Pro Gly Thr Leu Ile Ile Thr Glu Gly Thr Phe Pro Ser Pro Gln
    65                  70                  75

agc ggc ggc tat gat aac gcg ccg ggc att tgg agc gaa gaa cag att 288
Ser Gly Gly Tyr Asp Asn Ala Pro Gly Ile Trp Ser Glu Glu Gln Ile
80                  85                  90                  95

aaa gaa tgg acc aaa att ttt aaa gcg att cat gaa aac aaa agc ttt 336
Lys Glu Trp Thr Lys Ile Phe Lys Ala Ile His Glu Asn Lys Ser Phe
                100                 105                 110

gcg tgg gtg cag ctg tgg gtg ctg ggc tgg gcg gcg ttt ccg gat acc 384
Ala Trp Val Gln Leu Trp Val Leu Gly Trp Ala Ala Phe Pro Asp Thr
            115                 120                 125

ctg gcg cgc gat ggc ctg cgc tat gat agc gcg agc gat aac gtg tat 432
Leu Ala Arg Asp Gly Leu Arg Tyr Asp Ser Ala Ser Asp Asn Val Tyr
        130                 135                 140

atg aac gcg gaa cag gaa gaa aaa gcg aaa aaa gcg aac aac ccg cag 480
Met Asn Ala Glu Gln Glu Glu Lys Ala Lys Lys Ala Asn Asn Pro Gln
    145                 150                 155

cat agc att acc aaa gat gaa att aaa cag tat gtg aaa gaa tat gtg 528
His Ser Ile Thr Lys Asp Glu Ile Lys Gln Tyr Val Lys Glu Tyr Val
160                 165                 170                 175

cag gcg gcg aaa aac agc att gcg gcg ggc gcg gat ggc gtg gaa att 576
Gln Ala Ala Lys Asn Ser Ile Ala Ala Gly Ala Asp Gly Val Glu Ile
                180                 185                 190

cat agc gcg aac ggc tat ctg ctg aac cag ttt ctg gat ccg cat agc 624
His Ser Ala Asn Gly Tyr Leu Leu Asn Gln Phe Leu Asp Pro His Ser
            195                 200                 205

aac aac cgc acc gat gaa tat ggc ggc agc att gaa aac cgc gcg cgc 672
Asn Asn Arg Thr Asp Glu Tyr Gly Gly Ser Ile Glu Asn Arg Ala Arg
        210                 215                 220

ttt acc ctg gaa gtg gtg gat gcg gtg gtg gat gcg att ggc ccg gaa 720
Phe Thr Leu Glu Val Val Asp Ala Val Val Asp Ala Ile Gly Pro Glu
    225                 230                 235

aaa gtg ggc ctg cgc ctg agc ccg tat ggc gtg ttt aac agc atg agc 768
Lys Val Gly Leu Arg Leu Ser Pro Tyr Gly Val Phe Asn Ser Met Ser
240                 245                 250                 255

ggc ggc gcg gaa acc ggc att gtg gcg cag tat gcg tat gtg ctg ggc 816
Gly Gly Ala Glu Thr Gly Ile Val Ala Gln Tyr Ala Tyr Val Leu Gly
                260                 265                 270

gaa ctg gaa cgc cgc gcg aaa gcg ggc aaa cgc ctg gcg ttt gtg cat 864
Glu Leu Glu Arg Arg Ala Lys Ala Gly Lys Arg Leu Ala Phe Val His
            275                 280                 285

ctg gtg gaa ccg cgc gtg acc aac ccg ttt ctg acc gaa ggc gaa ggc 912
Leu Val Glu Pro Arg Val Thr Asn Pro Phe Leu Thr Glu Gly Glu Gly
        290                 295                 300

gaa tat aac ggc ggc agc aac aaa ttt gcg tat agc att tgg aaa ggc 960
Glu Tyr Asn Gly Gly Ser Asn Lys Phe Ala Tyr Ser Ile Trp Lys Gly
    305                 310                 315

ccg att att cgc gcg ggc aac ttt gcg ctg cat ccg gaa gtg gtg cgc 1008
Pro Ile Ile Arg Ala Gly Asn Phe Ala Leu His Pro Glu Val Val Arg
320                 325                 330                 335

gaa gaa gtg aaa gat ccg cgc acc ctg att ggc tat ggc cgc ttt ttt 1056
Glu Glu Val Lys Asp Pro Arg Thr Leu Ile Gly Tyr Gly Arg Phe Phe
                340                 345                 350

att agc aac ccg gat ctg gtg gat cgc ctg gaa aaa ggc ctg ccg ctg 1104
Ile Ser Asn Pro Asp Leu Val Asp Arg Leu Glu Lys Gly Leu Pro Leu
            355                 360                 365

aac aaa tat gat cgc gat acc ttt tat aaa atg agc gcg gaa ggc tat     1152
Asn Lys Tyr Asp Arg Asp Thr Phe Tyr Lys Met Ser Ala Glu Gly Tyr
        370                 375                 380

att gat tat ccg acc tat gaa gaa gcg ctg aaa ctg ggc tgg gat aaa     1200
Ile Asp Tyr Pro Thr Tyr Glu Glu Ala Leu Lys Leu Gly Trp Asp Lys
    385                 390                 395

aac atgacttaac tgcagccaag cttggctgtt ttggcggatg agagaagatt     1253
Asn
400

ttcagcctga tacagattaa atcagaacgc agaagcggtc tgataaaaca gaatttgcct     1313

ggcggcagta gcgcggtggt cccacctgac cccatgccga actcagaagt gaaacgccgt     1373

agcgccgatg gtagtgtggg gtctccccat gcgagagtag ggaactgcca ggcatcaaat     1433

aaaacgaaag gctcagtcga aagactgggc ctttcgtttt atctgttgtt tgtcggtgaa     1493

cgctctcctg agtaggacaa atccgccggg agcggatttg aacgttgcga agcaacggcc     1553

cggagggtgg cgggcaggac gcccgccata aactgccagg catcaaatta agcagaaggc     1613

catcctgacg gatggccttt ttgcgtttct acaaactctt ttgtttattt ttctaaatac     1673

attcaaatat gtatccgctc atgagacaat aaccctgata aatgcttcaa taatattgaa     1733

aaggaagagt atgagtattc aacatttccg tgtcgccctt attccctttt ttgcggcatt     1793

ttgccttcct gtttttgctc acccagaaac gctggtgaaa gtaaaagatg ctgaagatca     1853

gttgggtgca cgagtgggtt acatcgaact ggatctcaac agcggtaaga tccttgagag     1913

ttttcgcccc gaagaacgtt ttccaatgat gagcactttt aaagttctgc tatgtggcgc     1973

ggtattatcc cgtgttgacg ccgggcaaga gcaactcggt cgccgcatac actattctca     2033

gaatgacttg gttgagtact caccagtcac agaaaagcat cttacggatg gcatgacagt     2093

aagagaatta tgcagtgctg ccataaccat gagtgataac actgcggcca acttacttct     2153

gacaacgatc ggaggaccga aggagctaac cgcttttttg cacaacatgg gggatcatgt     2213

aactcgcctt gatcgttggg aaccggagct gaatgaagcc ataccaaacg acgagcgtga     2273

caccacgatg cctgtagcaa tggcaacaac gttgcgcaaa ctattaactg gcgaactact     2333

tactctagct tcccggcaac aattaataga ctggatggag gcggataaag ttgcaggacc     2393

acttctgcgc tcggcccttc cggctggctg gtttattgct gataaatctg gagccggtga     2453

gcgtgggtct cgcggtatca ttgcagcact ggggccagat ggtaagccct cccgtatcgt     2513

agttatctac acgacgggga gtcaggcaac tatggatgaa cgaaatagac agatcgctga     2573

gataggtgcc tcactgatta agcattggta actgtcagac caagtttact catatatact     2633

ttagattgat ttaaaacttc atttttaatt taaaaggatc taggtgaaga tcctttttga     2693

taatctcatg accaaaatcc cttaacgtga gttttcgttc cactgagcgt cagaccccgt     2753

agaaaagatc aaaggatctt cttgagatcc tttttttctg cgcgtaatct gctgcttgca     2813

aacaaaaaaa ccaccgctac cagcggtggt ttgtttgccg gatcaagagc taccaactct     2873

ttttccgaag gtaactggct tcagcagagc gcagatacca aatactgtcc ttctagtgta     2933

gccgtagtta ggccaccact tcaagaactc tgtagcaccg cctacatacc tcgctctgct     2993

aatcctgtta ccagtggctg ctgccagtgg cgataagtcg tgtcttaccg ggttggactc     3053

aagacgatag ttaccggata aggcgcagcg gtcgggctga acggggggtt cgtgcacaca     3113

gcccagcttg gagcgaacga cctacaccga actgagatac ctacagcgtg agctatgaga     3173

aagcgccacg cttcccgaag ggagaaaggc ggacaggtat ccggtaagcg gcagggtcgg     3233

aacaggagag cgcacgaggg agcttccagg gggaaacgcc tggtatcttt atagtcctgt     3293

cgggtttcgc cacctctgac ttgagcgtcg atttttgtga tgctcgtcag gggggcggag     3353

cctatggaaa aacgccagca acgcggcctt tttacggttc ctggcctttt gctggccttt 3413
tgctcacatg ttctttcctg cgttatcccc tgattctgtg gataaccgta ttaccgcctt 3473
tgagtgagct gataccgctc gccgcagccg aacgaccgag cgcagcgagt cagtgagcga 3533
ggaagcggaa gagcgcctga tgcggtattt tctccttacg catctgtgcg gtatttcaca 3593
ccgcatatat ggtgcactct cagtacaatc tgctctgatg ccgcatagtt aagccagtat 3653
acactccgct atcgctacgt gactgggtca tggctgcgcc ccgacacccg ccaacacccg 3713
ctgacgcgcc ctgacgggct tgtctgctcc cggcatccgc ttacagacaa gctgtgaccg 3773
tctccgggag ctgcatgtgt cagaggtttt caccgtcatc accgaaacgc gcgaggcagc 3833
tgcggtaaag ctcatcagcg tggtcgtgaa gcgattcaca gatgtctgcc tgttcatccg 3893
cgtccagctc gttgagtttc tccagaagcg ttaatgtctg gcttctgata aagcgggcca 3953
tgttaagggc ggttttttcc tgtttggtca cttgatgcct ccgtgtaagg gggaatttct 4013
gttcatgggg gtaatgatac cgatgaaacg agagaggatg ctcacgatac gggttactga 4073
tgatgaacat gcccggttac tggaacgttg tgagggtaaa caactggcgg tatggatgcg 4133
gcgggaccag agaaaaatca ctcagggtca atgccagcgc ttcgttaata cagatgtagg 4193
tgttccacag ggtagccagc agcatcctgc gatgcagatc cggaacataa tggtgcaggg 4253
cgctgacttc cgcgtttcca gactttacga aacacggaaa ccgaagacca ttcatgttgt 4313
tgctcaggtc gcagacgttt tgcagcagca gtcgcttcac gttcgctcgc gtatcggtga 4373
ttcattctgc taaccagtaa ggcaaccccg ccagcctagc cgggtcctca acgacaggag 4433
cacgatcatg cgcacccgtg gccaggaccc aacgctgccc gagatgcgcc gcgtgcggct 4493
gctggagatg gcggacgcga tggatatgtt ctgccaaggg ttggtttgcg cattcacagt 4553
tctccgcaag aattgattgg ctccaattct tggagtggtg aatccgttag cgaggtgccg 4613
ccggcttcca ttcaggtcga ggtggcccgg ctccatgcac cgcgacgcaa cgcggggagg 4673
cagacaaggt atagggcggc gcctacaatc catgccaacc cgttccatgt gctcgccgag 4733
gcggcataaa tcgccgtgac gatcagcggt ccagtgatcg aagttaggct ggtaagagcc 4793
gcgagcgatc cttgaagctg tccctgatgg tcgtcatcta cctgcctgga cagcatggcc 4853
tgcaacgcgg gcatcccgat gccgccggaa gcgagaagaa tcataatggg gaaggccatc 4913
cagcctcgcg tcgcgaacgc cagcaagacg tagcccagcg cgtcggccgc catgccggcg 4973
ataatggcct gcttctcgcc gaaacgtttg gtggcgggac cagtgacgaa ggcttgagcg 5033
agggcgtgca agattccgaa taccgcaagc gacaggccga tcatcgtcgc gctccagcga 5093
aagcggtcct cgccgaaaat gacccagagc gctgccggca cctgtcctac gagttgcatg 5153
ataaagaaga cagtcataag tgcggcgacg atagtcatgc cccgcgccca ccggaaggag 5213
ctgactgggt tgaaggctct caagggcatc ggtcgacgct ctcccttatg cgactcctgc 5273
attaggaagc agcccagtag taggttgagg ccgttgagca ccgccgccgc aaggaatggt 5333
gcatgcatcg atcaccacaa ttcagcaaat tgtgaacatc atcacgttca tctttccctg 5393
gttgccaatg gcccattttc ctgtcagtaa cgagaaggtc gcgaattcag gcgcttttta 5453
gactggtcgt aatgaacaat tcttaagaag gagatata 5491

<210> 7
<211> 400
<212> PRT
<213> Artificial

<220>
<223> Synthetic Construct

<400>  7

Met Pro Phe Val Lys Asp Phe Lys Pro Gln Ala Leu Gly Asp Thr Asn
1               5                   10                  15

Leu Phe Lys Pro Ile Lys Ile Gly Asn Asn Glu Leu Leu His Arg Ala
            20                  25                  30

Val Ile Pro Pro Leu Thr Arg Met Arg Ala Gln His Pro Gly Asn Ile
        35                  40                  45

Pro Asn Arg Asp Trp Ala Val Glu Tyr Tyr Ala Gln Arg Ala Gln Arg
    50                  55                  60

Pro Gly Thr Leu Ile Ile Thr Glu Gly Thr Phe Pro Ser Pro Gln Ser
65                  70                  75                  80

Gly Gly Tyr Asp Asn Ala Pro Gly Ile Trp Ser Glu Glu Gln Ile Lys
                85                  90                  95

Glu Trp Thr Lys Ile Phe Lys Ala Ile His Glu Asn Lys Ser Phe Ala
            100                 105                 110

Trp Val Gln Leu Trp Val Leu Gly Trp Ala Ala Phe Pro Asp Thr Leu
        115                 120                 125

Ala Arg Asp Gly Leu Arg Tyr Asp Ser Ala Ser Asp Asn Val Tyr Met
    130                 135                 140

Asn Ala Glu Gln Glu Glu Lys Ala Lys Lys Ala Asn Asn Pro Gln His
145                 150                 155                 160

Ser Ile Thr Lys Asp Glu Ile Lys Gln Tyr Val Lys Glu Tyr Val Gln
                165                 170                 175

Ala Ala Lys Asn Ser Ile Ala Ala Gly Ala Asp Gly Val Glu Ile His
            180                 185                 190

Ser Ala Asn Gly Tyr Leu Leu Asn Gln Phe Leu Asp Pro His Ser Asn
        195                 200                 205

Asn Arg Thr Asp Glu Tyr Gly Gly Ser Ile Glu Asn Arg Ala Arg Phe
    210                 215                 220

Thr Leu Glu Val Val Asp Ala Val Val Asp Ala Ile Gly Pro Glu Lys
225                 230                 235                 240

Val Gly Leu Arg Leu Ser Pro Tyr Gly Val Phe Asn Ser Met Ser Gly
                245                 250                 255

Gly Ala Glu Thr Gly Ile Val Ala Gln Tyr Ala Tyr Val Leu Gly Glu
            260                 265                 270

Leu Glu Arg Arg Ala Lys Ala Gly Lys Arg Leu Ala Phe Val His Leu
        275                 280                 285

Val Glu Pro Arg Val Thr Asn Pro Phe Leu Thr Glu Gly Glu Gly Glu
    290                 295                 300

Tyr Asn Gly Gly Ser Asn Lys Phe Ala Tyr Ser Ile Trp Lys Gly Pro
305                 310                 315                 320

Ile Ile Arg Ala Gly Asn Phe Ala Leu His Pro Glu Val Val Arg Glu
                325                 330                 335

Glu Val Lys Asp Pro Arg Thr Leu Ile Gly Tyr Gly Arg Phe Phe Ile
            340                 345                 350

Ser Asn Pro Asp Leu Val Asp Arg Leu Glu Lys Gly Leu Pro Leu Asn
        355                 360                 365

Lys Tyr Asp Arg Asp Thr Phe Tyr Lys Met Ser Ala Glu Gly Tyr Ile
    370                 375                 380

Asp Tyr Pro Thr Tyr Glu Glu Ala Leu Lys Leu Gly Trp Asp Lys Asn
385                 390                 395                 400

<210> 8
<211> 5474
<212> DNA
<213> Artificial

<220>
<223> Plasmid encoding OYE3

<220>
<221> CDS
<222> (4)..(1203)

<400> 8
cat atg ccg ttt gtg aaa ggc ttt gaa ccg att agc ctg cgc gat acc 48
Met Pro Phe Val Lys Gly Phe Glu Pro Ile Ser Leu Arg Asp Thr
1               5                   10                  15

aac ctg ttt gaa ccg att aaa att ggc aac acc cag ctg gcg cat cgc 96
Asn Leu Phe Glu Pro Ile Lys Ile Gly Asn Thr Gln Leu Ala His Arg
                20                  25                  30

gcg gtg atg ccg ccg ctg acc cgc atg cgc gcg acc cat ccg ggc aac 144
Ala Val Met Pro Pro Leu Thr Arg Met Arg Ala Thr His Pro Gly Asn
            35                  40                  45

att ccg aac aaa gaa tgg gcg gcg gtg tat tat ggc cag cgc gcg cag 192
Ile Pro Asn Lys Glu Trp Ala Ala Val Tyr Tyr Gly Gln Arg Ala Gln
        50                  55                  60

cgc ccg ggc acc atg att att acc gaa ggc acc ttt att agc ccg cag 240
Arg Pro Gly Thr Met Ile Ile Thr Glu Gly Thr Phe Ile Ser Pro Gln
    65                  70                  75

gcg ggc ggc tat gat aac gcg ccg ggc att tgg agc gat gaa cag gtg 288
Ala Gly Gly Tyr Asp Asn Ala Pro Gly Ile Trp Ser Asp Glu Gln Val
80                  85                  90                  95

gcg gaa tgg aaa aac att ttt ctg gcg att cat gat tgc cag agc ttt 336
Ala Glu Trp Lys Asn Ile Phe Leu Ala Ile His Asp Cys Gln Ser Phe
                100                 105                 110

gcg tgg gtg cag ctg tgg agc ctg ggc tgg gcg agc ttt ccg gat gtg 384
Ala Trp Val Gln Leu Trp Ser Leu Gly Trp Ala Ser Phe Pro Asp Val
            115                 120                 125

ctg gcg cgc gat ggc ctg cgc tat gat tgc gcg agc gat cgc gtg tat 432
Leu Ala Arg Asp Gly Leu Arg Tyr Asp Cys Ala Ser Asp Arg Val Tyr
        130                 135                 140

atg aac gcg acc ctg cag gaa aaa gcg aaa gat gcg aac aac ctg gaa 480
Met Asn Ala Thr Leu Gln Glu Lys Ala Lys Asp Ala Asn Asn Leu Glu
    145                 150                 155

cat agc ctg acc aaa gat gat att aaa cag tat att aaa gat tat att 528
His Ser Leu Thr Lys Asp Asp Ile Lys Gln Tyr Ile Lys Asp Tyr Ile
160                 165                 170                 175

cat gcg gcg aaa aac agc att gcg gcg ggc gcg gat ggc gtg gaa att 576

His Ala Ala Lys Asn Ser Ile Ala Ala Gly Ala Asp Gly Val Glu Ile
                180                 185                 190

cat agc gcg aac ggc tat ctg ctg aac cag ttt ctg gat ccg cat agc 624
His Ser Ala Asn Gly Tyr Leu Leu Asn Gln Phe Leu Asp Pro His Ser
            195                 200                 205

aac aaa cgc acc gat gaa tat ggc ggc acc att gaa aac cgc gcg cgc 672
Asn Lys Arg Thr Asp Glu Tyr Gly Gly Thr Ile Glu Asn Arg Ala Arg
        210                 215                 220

ttt acc ctg gaa gtg gtg gat gcg ctg att gaa acc att ggc ccg gaa 720
Phe Thr Leu Glu Val Val Asp Ala Leu Ile Glu Thr Ile Gly Pro Glu
    225                 230                 235

cgc gtg ggc ctg cgc ctg agc ccg tat ggc acc ttt aac agc atg agc 768
Arg Val Gly Leu Arg Leu Ser Pro Tyr Gly Thr Phe Asn Ser Met Ser
240                 245                 250                 255

ggc ggc gcg gaa ccg ggc att att gcg cag tat agc tat gtg ctg ggc 816
Gly Gly Ala Glu Pro Gly Ile Ile Ala Gln Tyr Ser Tyr Val Leu Gly
                260                 265                 270

gaa ctg gaa aaa cgc gcg aaa gcg ggc aaa cgc ctg gcg ttt gtg cat 864
Glu Leu Glu Lys Arg Ala Lys Ala Gly Lys Arg Leu Ala Phe Val His
            275                 280                 285

ctg gtg gaa ccg cgc gtg acc gat ccg agc ctg gtg gaa ggc gaa ggc 912
Leu Val Glu Pro Arg Val Thr Asp Pro Ser Leu Val Glu Gly Glu Gly
        290                 295                 300

gaa tat agc gaa ggc acc aac gat ttt gcg tat agc att tgg aaa ggc 960
Glu Tyr Ser Glu Gly Thr Asn Asp Phe Ala Tyr Ser Ile Trp Lys Gly
    305                 310                 315

ccg att att cgc gcg ggc aac tat gcg ctg cat ccg gaa gtg gtg cgc 1008
Pro Ile Ile Arg Ala Gly Asn Tyr Ala Leu His Pro Glu Val Val Arg
320                 325                 330                 335

gaa cag gtg aaa gat ccg cgc acc ctg att ggc tat ggc cgc ttt ttt 1056
Glu Gln Val Lys Asp Pro Arg Thr Leu Ile Gly Tyr Gly Arg Phe Phe
                340                 345                 350

att agc aac ccg gat ctg gtg tat cgc ctg gaa gaa ggc ctg ccg ctg 1104
Ile Ser Asn Pro Asp Leu Val Tyr Arg Leu Glu Glu Gly Leu Pro Leu
            355                 360                 365

aac aaa tat gat cgc agc acc ttt tat acc atg agc gcg gaa ggc tat 1152
Asn Lys Tyr Asp Arg Ser Thr Phe Tyr Thr Met Ser Ala Glu Gly Tyr
        370                 375                 380

acc gat tat ccg acc tat gaa gaa gcg gtg gat ctg ggc tgg aac aaa 1200
Thr Asp Tyr Pro Thr Tyr Glu Glu Ala Val Asp Leu Gly Trp Asn Lys
    385                 390                 395

aac aagcttggct gttttggcgg atgagagaag attttcagcc tgatacagat 1253
Asn
400

taaatcagaa cgcagaagcg gtctgataaa acagaatttg cctggcggca gtagcgcggt 1313

ggtcccacct gaccccatgc cgaactcaga agtgaaacgc cgtagcgccg atggtagtgt 1373

ggggtctccc catgcgagag tagggaactg ccaggcatca aataaaacga aaggctcagt 1433

cgaaagactg ggcctttcgt tttatctgtt gtttgtcggt gaacgctctc ctgagtagga 1493

caaatccgcc gggagcggat ttgaacgttg cgaagcaacg gcccggaggg tggcgggcag 1553

gacgcccgcc ataaactgcc aggcatcaaa ttaagcagaa ggccatcctg acggatggcc 1613

tttttgcgtt tctacaaact cttttgttta tttttctaaa tacattcaaa tatgtatccg 1673

ctcatgagac aataaccctg ataaatgctt caataatatt gaaaaggaag agtatgagta 1733

ttcaacattt ccgtgtcgcc cttattccct tttttgcggc attttgcctt cctgtttttg 1793

ctcacccaga aacgctggtg aaagtaaaag atgctgaaga tcagttgggt gcacgagtgg 1853

gttacatcga actggatctc aacagcggta agatccttga gagttttcgc cccgaagaac 1913

gttttccaat gatgagcact tttaaagttc tgctatgtgg cgcggtatta tcccgtgttg 1973
acgccgggca agagcaactc ggtcgccgca tacactattc tcagaatgac ttggttgagt 2033
actcaccagt cacagaaaag catcttacgg atggcatgac agtaagagaa ttatgcagtg 2093
ctgccataac catgagtgat aacactgcgg ccaacttact tctgacaacg atcggaggac 2153
cgaaggagct aaccgctttt ttgcacaaca tgggggatca tgtaactcgc cttgatcgtt 2213
gggaaccgga gctgaatgaa gccataccaa acgacgagcg tgacaccacg atgcctgtag 2273
caatggcaac aacgttgcgc aaactattaa ctggcgaact acttactcta gcttcccggc 2333
aacaattaat agactggatg gaggcggata aagttgcagg accacttctg cgctcggccc 2393
ttccggctgg ctggtttatt gctgataaat ctggagccgg tgagcgtggg tctcgcggta 2453
tcattgcagc actggggcca gatggtaagc cctcccgtat cgtagttatc tacacgacgg 2513
ggagtcaggc aactatggat gaacgaaata gacagatcgc tgagataggt gcctcactga 2573
ttaagcattg gtaactgtca gaccaagttt actcatatat actttagatt gatttaaaac 2633
ttcattttta atttaaaagg atctaggtga agatcctttt tgataatctc atgaccaaaa 2693
tcccttaacg tgagttttcg ttccactgag cgtcagaccc cgtagaaaag atcaaaggat 2753
cttcttgaga tccttttttt ctgcgcgtaa tctgctgctt gcaaacaaaa aaaccaccgc 2813
taccagcggt ggtttgtttg ccggatcaag agctaccaac tctttttccg aaggtaactg 2873
gcttcagcag agcgcagata ccaaatactg tccttctagt gtagccgtag ttaggccacc 2933
acttcaagaa ctctgtagca ccgcctacat acctcgctct gctaatcctg ttaccagtgg 2993
ctgctgccag tggcgataag tcgtgtctta ccgggttgga ctcaagacga tagttaccgg 3053
ataaggcgca gcggtcgggc tgaacggggg gttcgtgcac acagcccagc ttggagcgaa 3113
cgacctacac cgaactgaga tacctacagc gtgagctatg agaaagcgcc acgcttcccg 3173
aagggagaaa ggcggacagg tatccggtaa gcggcagggt cggaacagga gagcgcacga 3233
gggagcttcc agggggaaac gcctggtatc tttatagtcc tgtcgggttt cgccacctct 3293
gacttgagcg tcgatttttg tgatgctcgt caggggggcg gagcctatgg aaaaacgcca 3353
gcaacgcggc ctttttacgg ttcctggcct tttgctggcc ttttgctcac atgttctttc 3413
ctgcgttatc ccctgattct gtggataacc gtattaccgc ctttgagtga gctgataccg 3473
ctcgccgcag ccgaacgacc gagcgcagcg agtcagtgag cgaggaagcg gaagagcgcc 3533
tgatgcggta ttttctcctt acgcatctgt gcggtatttc acaccgcata tatggtgcac 3593
tctcagtaca atctgctctg atgccgcata gttaagccag tatacactcc gctatcgcta 3653
cgtgactggg tcatggctgc gccccgacac ccgccaacac ccgctgacgc gccctgacgg 3713
gcttgtctgc tcccggcatc cgcttacaga caagctgtga ccgtctccgg gagctgcatg 3773
tgtcagaggt tttcaccgtc atcaccgaaa cgcgcgaggc agctgcggta aagctcatca 3833
gcgtggtcgt gaagcgattc acagatgtct gcctgttcat ccgcgtccag ctcgttgagt 3893
ttctccagaa gcgttaatgt ctggcttctg ataaagcggg ccatgttaag ggcggttttt 3953
tcctgtttgg tcacttgatg cctccgtgta agggggaatt tctgttcatg ggggtaatga 4013
taccgatgaa acgagagagg atgctcacga tacgggttac tgatgatgaa catgcccggt 4073
tactggaacg ttgtgagggt aaacaactgg cggtatggat gcggcgggac cagagaaaaa 4133
tcactcaggg tcaatgccag cgcttcgtta atacagatgt aggtgttcca cagggtagcc 4193
agcagcatcc tgcgatgcag atccggaaca taatggtgca gggcgctgac ttccgcgttt 4253
ccagacttta cgaaacacgg aaaccgaaga ccattcatgt tgttgctcag gtcgcagacg 4313
ttttgcagca gcagtcgctt cacgttcgct cgcgtatcgg tgattcattc tgctaaccag 4373

taaggcaacc ccgccagcct agccgggtcc tcaacgacag gagcacgatc atgcgcaccc 4433

gtggccagga cccaacgctg cccgagatgc gccgcgtgcg gctgctggag atggcggacg 4493

cgatggatat gttctgccaa gggttggttt gcgcattcac agttctccgc aagaattgat 4553

tggctccaat tcttggagtg gtgaatccgt tagcgaggtg ccgccggctt ccattcaggt 4613

cgaggtggcc cggctccatg caccgcgacg caacgcgggg aggcagacaa ggtatagggc 4673

ggcgcctaca atccatgcca acccgttcca tgtgctcgcc gaggcggcat aaatcgccgt 4733

gacgatcagc ggtccagtga tcgaagttag gctggtaaga gccgcgagcg atccttgaag 4793

ctgtccctga tggtcgtcat ctacctgcct ggacagcatg gcctgcaacg cgggcatccc 4853

gatgccgccg gaagcgagaa gaatcataat ggggaaggcc atccagcctc gcgtcgcgaa 4913

cgccagcaag acgtagccca gcgcgtcggc cgccatgccg gcgataatgg cctgcttctc 4973

gccgaaacgt ttggtggcgg gaccagtgac gaaggcttga gcgagggcgt gcaagattcc 5033

gaataccgca agcgacaggc cgatcatcgt cgcgctccag cgaaagcggt cctcgccgaa 5093

aatgacccag agcgctgccg gcacctgtcc tacgagttgc atgataaaga agacagtcat 5153

aagtgcggcg acgatagtca tgccccgcgc ccaccggaag gagctgactg ggttgaaggc 5213

tctcaagggc atcggtcgac gctctccctt atgcgactcc tgcattagga agcagcccag 5273

tagtaggttg aggccgttga gcaccgccgc cgcaaggaat ggtgcatgca tcgatcacca 5333

caattcagca aattgtgaac atcatcacgt tcatctttcc ctggttgcca atggcccatt 5393

ttcctgtcag taacgagaag gtcgcgaatt caggcgcttt ttagactggt cgtaatgaac 5453

aattcttaag aaggagatat a 5474

<210> 9
<211> 400
<212> PRT
<213> Artificial

<220>
<223> Synthetic Construct

<400> 9

Met Pro Phe Val Lys Gly Phe Glu Pro Ile Ser Leu Arg Asp Thr Asn
1               5                   10                  15

Leu Phe Glu Pro Ile Lys Ile Gly Asn Thr Gln Leu Ala His Arg Ala
            20                  25                  30

Val Met Pro Pro Leu Thr Arg Met Arg Ala Thr His Pro Gly Asn Ile
        35                  40                  45

Pro Asn Lys Glu Trp Ala Ala Val Tyr Tyr Gly Gln Arg Ala Gln Arg
    50                  55                  60

Pro Gly Thr Met Ile Ile Thr Glu Gly Thr Phe Ile Ser Pro Gln Ala
65                  70                  75                  80

Gly Gly Tyr Asp Asn Ala Pro Gly Ile Trp Ser Asp Glu Gln Val Ala
                85                  90                  95

Glu Trp Lys Asn Ile Phe Leu Ala Ile His Asp Cys Gln Ser Phe Ala
            100                 105                 110

Trp Val Gln Leu Trp Ser Leu Gly Trp Ala Ser Phe Pro Asp Val Leu
        115                 120                 125

Ala Arg Asp Gly Leu Arg Tyr Asp Cys Ala Ser Asp Arg Val Tyr Met
    130                 135                 140

Asn Ala Thr Leu Gln Glu Lys Ala Lys Asp Ala Asn Asn Leu Glu His
145                 150                 155                 160

Ser Leu Thr Lys Asp Asp Ile Lys Gln Tyr Ile Lys Asp Tyr Ile His
                165                 170                 175

Ala Ala Lys Asn Ser Ile Ala Ala Gly Ala Asp Gly Val Glu Ile His
            180                 185                 190

Ser Ala Asn Gly Tyr Leu Leu Asn Gln Phe Leu Asp Pro His Ser Asn
        195                 200                 205

Lys Arg Thr Asp Glu Tyr Gly Gly Thr Ile Glu Asn Arg Ala Arg Phe
    210                 215                 220

Thr Leu Glu Val Val Asp Ala Leu Ile Glu Thr Ile Gly Pro Glu Arg
225                 230                 235                 240

Val Gly Leu Arg Leu Ser Pro Tyr Gly Thr Phe Asn Ser Met Ser Gly
                245                 250                 255

Gly Ala Glu Pro Gly Ile Ile Ala Gln Tyr Ser Tyr Val Leu Gly Glu
            260                 265                 270

Leu Glu Lys Arg Ala Lys Ala Gly Lys Arg Leu Ala Phe Val His Leu
        275                 280                 285

Val Glu Pro Arg Val Thr Asp Pro Ser Leu Val Glu Gly Glu Gly Glu
    290                 295                 300

Tyr Ser Glu Gly Thr Asn Asp Phe Ala Tyr Ser Ile Trp Lys Gly Pro
305                 310                 315                 320

Ile Ile Arg Ala Gly Asn Tyr Ala Leu His Pro Glu Val Val Arg Glu
                325                 330                 335

Gln Val Lys Asp Pro Arg Thr Leu Ile Gly Tyr Gly Arg Phe Phe Ile
            340                 345                 350

Ser Asn Pro Asp Leu Val Tyr Arg Leu Glu Glu Gly Leu Pro Leu Asn
        355                 360                 365

Lys Tyr Asp Arg Ser Thr Phe Tyr Thr Met Ser Ala Glu Gly Tyr Thr
    370                 375                 380

Asp Tyr Pro Thr Tyr Glu Glu Ala Val Asp Leu Gly Trp Asn Lys Asn
385                 390                 395                 400

## Beschreibung

Die Erfindung betrifft ein Verfahren zur enzymatischen Reduktion von Alkinderivaten.

### Stand der Technik

Bisher sind keine Verfahren zur enzymatischen Reduktion von Alkinderivaten bekannt, die kein ATP benötigen.

Es bestand die Aufgabe, ein Verfahren zur enzymatischen Herstellung Alkinderivaten bereitzustellen, das kein ATP benötigt.

### Kurzfassung der Erfindung

Obige Aufgabe wurde gelöst durch Verwendung der Reduktasen OYE 1, 2 und 3 und funktionaler Äquivalente davon zur Reduktion von Alkinderivaten der allgemeinen Formel (1).

### Figurenbeschreibung

In den beiliegenden Figuren zeigt
Figur 1 die Biotransformation von 20 mM 4-Phenyl-3-butin-2-on mit OYE 1-3 in Gegenwart von NADPH (links) oder NADH (rechts), EDTA und Isopropanol bei pH 6.8 und 30°C nach 1h. Die Enzyme wurden in *E. coli* TG10+ überexprimiert, TG10+ selbst diente als Kontrolle;

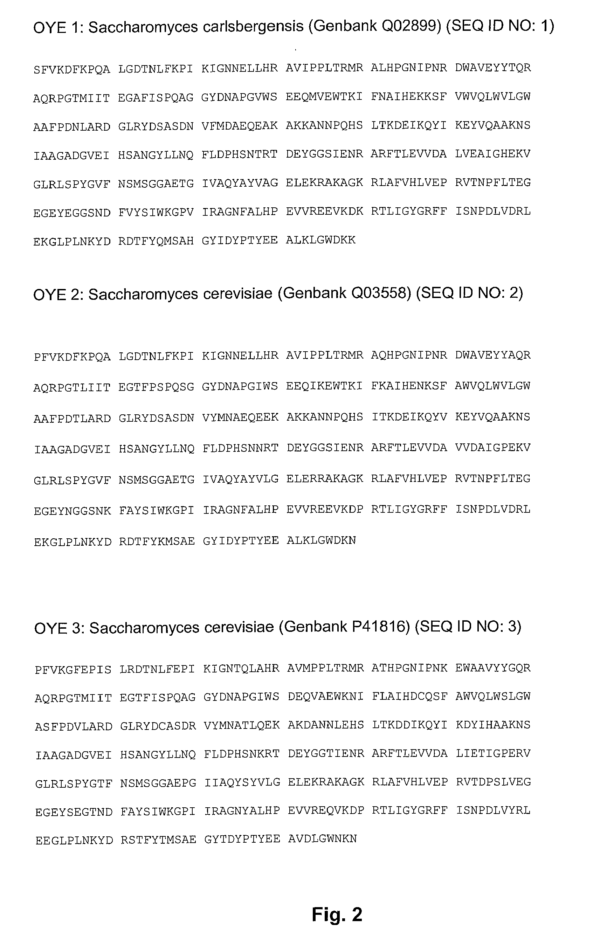

Figur 2 die Aminosäuresequenzen der verwendeten Enzyme OYE 1, 2 und 3;
Figur 3 die Plasmidkarte von pAgro4;
Figur 4 die Plasmidkarte von pHSG575';
Figur 5 die Plasmidkarte von pDHE1658 OYE1;
Figur 6 die Plasmidkarte von pDHE1658 OYE2; und
Figur 7 die Plasmidkarte von pDHE1658 OYE3.

### Detaillierte Beschreibung der Erfindung

Die vorliegende Erfindung betrifft ein Verfahren zur enzymatischen Herstellung von Alkenon-Derivaten der allgemeinen Formel (2) aus α,β- ungesättigten Alkinon-Derivaten der allgemeinen Formel (1) 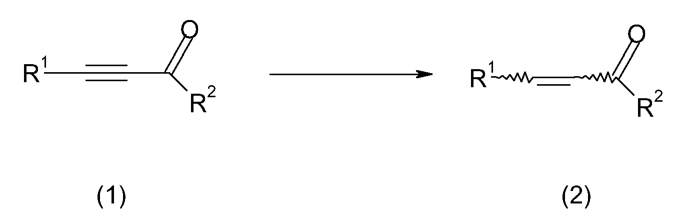 worin
R¹ für H, C₁-C₆- Alkyl, C₂-C₆- Alkenyl, oder einen gegebenenfalls substituierten carbo- oder hetrocyclischen aromatischen oder nicht aromatischen Ring steht, und
R² für H, C₁-C₆- Alkyl oder C₂-C₆-Alkenyl steht,
durch Reduktion einer Verbindung der Formel (1) in Gegenwart einer Reduktase
   - (i): umfassend wenigstens eine der Polypeptidsequenzen SEQ ID NO:1, 2, 3, 5, 7 oder 9 oder
   - (ii): mit einer funktional äquivalenten Polypeptidsequenz, die mindestens 80% Sequenzidentität mit SEQ ID NO:1, 2, 3, 5, 7 oder 9 aufweist,
erfolgt.

Vorzugsweise wird erfindungsgemäß insbesondere die E-Konfiguration von Verbindungen der Formel 2 bereitgestellt, 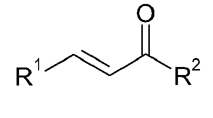 worin R¹ und R² die oben angegebenen Bedeutungen besitzen. Insbesondere liegen die Verbindungen der Formel 2 zu mehr als 50%, insbesondere mehr als 60, 70 oder 80 % vorzugsweise aber zu mehr als 90%, wie z.B. 95 bis 99%, insbesondere zu etwa 100% in der E-Konfiguration vor.

Grundsätzlich ist das erfindungsgemäße Verfahren sowohl mit gereinigten oder angereicherten Enzym selbst als auch mit Mikroorganismen, welche dieses Enzym natürlich oder rekombinant exprimieren, oder mit davon abgeleiteten Zellhomogenaten durchführbar.

Werden keine anderen Angaben gemacht, so bedeutet:
- C₁-C₆-Alkyl insbesondere Methyl, Ethyl, Propyl, Butyl, Pentyl oder Hexyl, sowie die entsprechenden ein- oder mehrfach verzweigte Analoga, wie i-Propyl, i-Butyl, sec.-Butyl, tert.-Butyl, i-Pentyl oder Neopentyl; wobei insbesondere die genannten C₁-C₄-Alkylreste bevorzugt sind;
- C₂-C₆-Alkenyl insbesondere die einfach ungesättigten Analoga der oben genannten Alkylreste mit 2 bis 6 Kohlenstoffatomen, wobei insbesondere die entsprechenden C₂-C₄-Alkenylreste bevorzugt sind.
- Carbo- und heterocyclische aromatische oder nichtaromatische Ringe insbesondere gegebenenfalls kondensierte Ringe mit 3 bis 12 Kohlenstoffatomen und gegebenenfalls 1 bis 4 Heteroatomen, wie N, S und O, insbesondere N oder O. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; Phenyl und Naphthyl; sowie 5- bis 7-gliedrige gesättigte oder ungesättigte heterocyclische Reste mit 1 bis 4 Heteroatomen, die ausgewählt sind unter O, N und S, wobei der Heterocyclus gegebenenfalls mit einem weitern Heterocyclus oder Carbocyclus kondensiert sein kann. Insbesondere sind zu nennen heterocyclische Reste abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin. Gegebenenfalls können die cyclischen Reste, aber auch die oben genannten Alkyl-und Alkenylreste ein- oder mehrfach, wie z. B. 1-, 2- oder 3-fach, substituiert sein. Als Beispiel für geeignete Substituierte sind zu nennen: Halogen, insbesondere F, Cl, Br; - OH, -SH, -NO₂, -NH₃, -SO₃H, C₁-C₄-Alkyl und C₂-C₄-Alkenyl, C₁-C₄ -Alkoxy; und Hydro-xy-C₁-C₄-Alkyl; wobei die Alkyl- und Alenyl-Reste wie oben definiert sind und die Alkoxy-Reste von den oben definierten korrespondierenden Alkylresten abgeleitet sind.

Das erfindungsgemäße Verfahren kann insbesondere mit Alkinen der allgemeinen Formel (1) durchgeführt werden, in denen R¹ für C₁-C₄- Alkyl in verzweigter und unverzweigter Form, C₂-C₆ Alkenyl in verzweigter und unverzweigter Form oder gegebenenfalls substituiertes Phenyl steht.

Besonders geeignete Substrate für das erfindungsgemäße Verfahren sind solche Alkine der allgemeinen Formel (1), bei denen R¹ für gegebenenfalls substituiertes Phenyl steht und R² für CH₃ steht.

Die erfindungsgemäß verwendeten Reduktasen reduzieren gelegentlich teilweise neben der Dreifachbindung in α,β-Position zur Carbonylfunktion auch die Carbonylfunktion selbst, wodurch dann der entsprechende Alkohol gebildet wird. Ebenso können die Alkene teilweise zum korrespondierenden Alkan weitereduziert werden.

Für das erfindungsgemäße Verfahren geeignete Reduktasen sind all diejenigen Enzyme, die in der Lage sind, 4-Phenyl-3-butin-2-on in einer NAD(P)H abhängigen, und vorzugsweise ATP-unabhängigen, Reaktion zu E-4-Phenyl-3-buten-2-on zu reduzieren. Diese Reaktion wird im Folgenden auch Modellreaktion genannt. 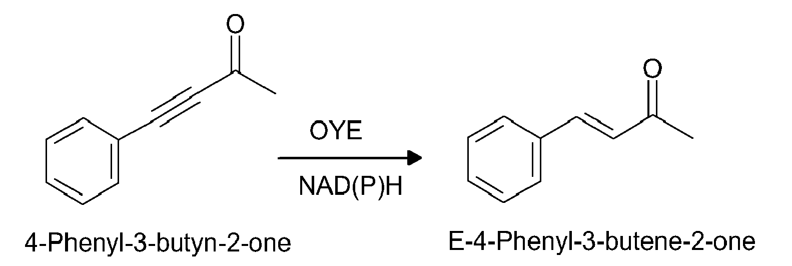 Weiterhin besitzen die für das erfindungsgemäße Verfahren geeigneten Reduktasen (welche zuweilen auch als Enoat-Reduktasen bezeichnet werden) eine Polypeptidsequenz gemäss SEQ ID NO:1, 2, 3, 5, 7 oder 9 oder eine Polypeptidsequenz, die mindestens 80%, wie z.B. mindestens 90%, oder mindestens 95% und insbesondere mindestens 97%, 98% oder 99% Sequenzidentität mit SEQ ID NO: 1, 2, 3, 5, 7 oder 9 aufweist.

Ein Polypeptid mit der SEQ ID NO:1 ist bekannt unter der Bezeichnung OYE1 aus Saccharomyces carlsbergensis (Genbank Q02899).

Ein Polypeptid mit der SEQ ID NO:2 wird kodiert vom OYE2 Gen aus Bäckerhefe (Saccharomyces cerevisiae Genlocus YHR179W) (Genbank Q03558).

Ein Polypeptid mit der SEQ ID NO:3 wird kodiert vom OYE3 Gen aus Bäckerhefe (Saccharomyces cerevisiae Genlocus YPL171C) (Genbank P 41816).

Die Sequenzen gemäß SEQ ID NO: 5, 7 und 9 entsprechen SEQ ID NO: 1, 2 und 3 und unterscheiden sich davon lediglich durch einen zusätzlichen N-terminalen Methioninrest.

Die Ermittlung der Sequenzidentität soll für die hier beschriebenen Zwecke durch das Computerprogramm "GAP" der Genetics Computer Group (GCG) der University of Wisconsin erfolgen, wobei die Version 10.3 unter Verwendung der von GCG empfohlenen Standardparameter zum Einsatz kommen soll.

Solche Reduktasen können ausgehend von SEQ ID NO: 1, 2, 3, 5, 7 oder 9 durch dem Fachmann bekannte gezielte oder randomisierte Mutageneseverfahren erhalten werden. Alternativ kann jedoch auch in Mikroorganismen , bevorzugt in solchen der Gattungen *Alishewanella, Alterococcus, Aquamonas, Aranicola, Arsenophonus, Azotivirga, Brenneria, Buchnera (aphid P-endosymbionts), Budvicia, Buttiauxella, Candidatus Phlomobacter, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia* oder *Yokenella,* nach Reduktasen gesucht werden, die die o.g. Modellreaktion katalysieren und deren Aminosäuresequenz die geforderte Sequenzidentität zu SEQ ID NO: 1, 2, 3, 5, 7 oder 9 bereits aufweist oder durch Mutageneseverfahren erhalten wird.

Die Reduktase kann in gereinigter oder teilweise gereinigter Form oder auch in Form des Mikroorganismus selbst verwendet werden. Verfahren zur Gewinnung und Aufreinigung von Dehydrogenasen aus Mikroorganismen sind dem Fachmann hinreichend bekannt.

Bevorzugt erfolgt die die enantioselektive Reduktion mit der Reduktase in Gegenwart eines geeigneten Cofaktors (auch als Cosubstrat bezeichnet). Als Cofaktoren für die Reduktion des Ketons dient üblicherweise NADH und/oder NADPH. Daneben können Reduktasen als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Bevorzugt erfolgt die enantioselektive Reduktion mit der Reduktase außerdem in Gegenwart eines geeigneten Reduktionsmittels, welches den im Verlauf der Reduktion oxidierten Cofaktor regeneriert. Beispiele für geeignete Reduktionsmittels sind Zucker, insbesondere Hexosen, wie Glucose, Mannose, Fructose, und/oder oxidierbare Alkohole, insbesondere Ethanol, Propanol oder Isopropanol, sowie Formiat, Phosphit oder molekularer Wasserstoff. Zur Oxidation des Reduktionsmittels und damit verbunden zur Regeneration des Coenzyms kann eine zweite Dehydrogenase, wie z.B. Glucose-dehydrogenase bei Verwendung von Glucose als Reduktionsmittel oder Formiat-Dehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Reduktase-Stamm erfolgen.

Eine bevorzugte Ausführungsform des beanspruchten Verfahrens ist die Regenerierung der Cofaktoren durch ein enzymatisches System, bei dem eine zweite Dehydrogenase, besonders bevorzugt eine Glucosedehydrogenase, verwendet wird.

Weiterhin kann es zweckmäßig sein, weitere die Reduktion fördernde Zusätze, wie z. B. Metallsalze oder Cheletbildner, wie. z. B. EDTA, zu zusetzten.

Die erfindungsgemäß verwendeten Reduktasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Crosslinking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Umsetzung kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Beispielsweise kann die Reduktion mit der Reduktase in einem wässrig-organischen Reaktionsmedium, wie z. B. Wasser/Isopropanol, in beliebigem Mischungsverhältnis wie z.B. 1:99 bis 99:1 oder 10: 90 bis 90:10, oder einem wässrigen Reaktionsmedium durchgeführt werden.

Das Substrat (1) wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Reduktase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Zur Durchführung kann man beispielsweise das Substrat (1) mit der Reduktase, dem Lösungsmittel und gegebenenfalls den Coenzymen, gegebenenfalls einer zweiten Dehydrogenase zur Regenerierung des Coenzyms und/oder weiteren Reduktionsmitteln vorlegen und das Gemisch durchmischen, z. B. durch Rühren oder Schütteln. Es ist aber auch möglich, die Reduktase in einem Reaktor, beispielsweise in einer Säule, zu immobilisieren, und durch den Reaktor eine das Substrat und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist.

Dabei wird die Dreifachbindung in α,β-Position zur Carbonylfunktion zur Doppelbindung reduziert; gelegentlich erfolgt auch eine Reduktion der Carbonylfunktion selbst zur Alkoholfunktion. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Substrat führen. Das Fortschreiten der Reaktion, d. h. die sequentielle Reduktion der Doppelbindung kann dabei durch übliche Methoden wie Gaschromatographie oder Hochdruckflüssigkeitschromatographie verfolgt werden.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die die Modellreaktion katalysieren und die mindestens 20 %, bevorzugt 50 %, besonders bevorzugt 75 %, ganz besonders bevorzugt 90 % der Aktivität eines Enzyms, umfassend eine der unter SEQ ID NO:1, 2 oder 3 aufgeführten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | lle; Leu |

"Funktionale Äquivalente" im obigen Sinne sind auch "Präkursoren" der beschriebenen Polypeptide sowie "funktionale Derivate".

"Präkursoren" sind dabei natürliche oder synthetische Vorstufen der Polypeptide mit oder ohne der gewünschten biologischen Aktiviät.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; lke et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Gegenstand der Erfindung sind weiterhin Nukleinsäuresequenzen (einzel- und doppelsträngige DNA- und RNA-Sequenzen, wie z.B. cDNA und mRNA), die für ein Enzym mit erfindungsgemäßer Reduktase -Aktivität kodieren. Bevorzugt sind Nukleinsäuresequenzen, welche z.B. für Aminosäuresequenzen gemäß SEQ ID NO:1, 2 oder 3 charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen enzymatischen Reduktionsverfahrens:

Die erfindungsgemäß eingesetzten Reduktasen können im erfindungsgemäßen Verfahren als freies oder immobilisiertes Enzym verwendet werden.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten. min. 98 %ee erreicht.

Für das erfindungsgemäße Verfahren können wachsende Zellen verwendet werden, die für die Reduktase kodierenden Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen, ohne sie jedoch einzuschränken. Hierbei wird auf beiliegende Abbildungen Bezug genommen, dabei zeigt:

### Experimenteller Teil

### Beispiel 1: Herstellung der Beschreibung der Reduktase exprimierenden E. coli TG10⁺ Transformanten und der korrespondierenden Transformationskonstrukte

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikrorganismen, Vermehrung von Phagen und Sequenzanalyse rekombinanter DNA, wie bei Sambrook et al. (1989) a.a.O. beschrieben durchgeführt werden.

Der rekombinante Escherichia-coli-Stamm TG10⁺(OYE) wurde wie folgt konstruiert:

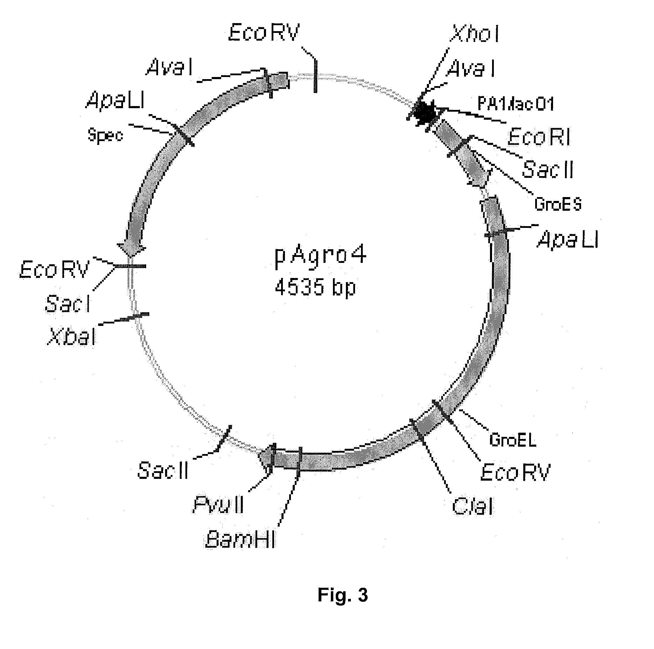

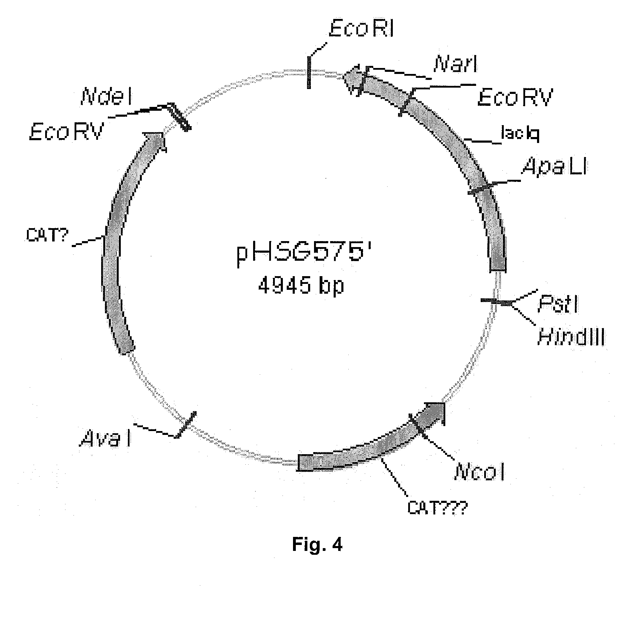

E.coli TG10 ist von E. coli TG1 (Stratagene) abgeleitet. TG10 ist ein tetracyclinresistenter und Rhamnose auxotropher Stamm. TG10⁺ ist von TG10 durch Einführung der folgenden Plasmide abgeleitet:
a) pAgro (Chaperon + Streptomycinresistenz) (vgl. Figur 3) und
b) pHSG575 (Chaperon + Chloramphenicolresistenz) (vgl. Figur 4).

pAgro4 ist ein Abkömmling der pZ-Vektoren mit groELS-Chaperoningenen aus E. coli. pZ wiederum ist ein Derivat des pACYC-Plasmids. pAgro4 ist beispielsweise beschrieben in Nucleic Acids Res., 1997, 25, 1203, Mol.Microbiol., 2001, 40, 397.
pHSG575ist ein Abkömmling von pSC101 mit laclq-Repressorgen aus E. coli und beispilelsweise beschrieben in Gene, 1987, 61, 63, Mol.Microbiol., 2001, 40, 397
TG10⁺(OYE) wurde von TG10⁺ durch Einführung eines weiteren Plasmids abgeleitet:
pDHE 1650 (langsamer Rhamnosepromoter + Ampicillinresistenz + ein oye-Gen).
pDHE1650 ist ein Abkömmling von pJOE2702 bei dem Gene für die ,old yellow enzymes' zwischen die Ndel und Pstl- bzw. Hindlll-Schnittstellen von pJOE2702 kloniert wurde. pJOE2702 wiederum wurde hergestellt, indem die rhaB Sequenz aus E. coli JM109 amplifiziert und in die Sphl bzw. EcoRI Schnittstellen des pBR322-Abkömmlings pBTAC1 kloniert wurde. Die Ribosomenbindestelle von pET11a sowie eine Ndel Schnittstelle wurden mit synthetischen Oligonukleotiden gewonnen und zwischen die BamHl / EcoRl Schnittstellen von pBTAC1 gesetzt. Das Plasmid ist beispielsweise beschrieben in Methods Enzymol., 1992, 216, 457, Mol.Microbiol., 1996, 21,1037.

### Auf obige Literaturstellen wird hiermit ausdrücklich Bezug genommen

Die folgenden Old Yellow Enzyme (oye) Gene wurde kloniert:
a) oye 1: Saccaromyces carlsbergensis (Genbank Q02899)
b) oye 2: Saccaromyces cerevisiae (Genbank Q03558)
c) oye 3: Saccaromyces cerevisiae (Genbank P41816).

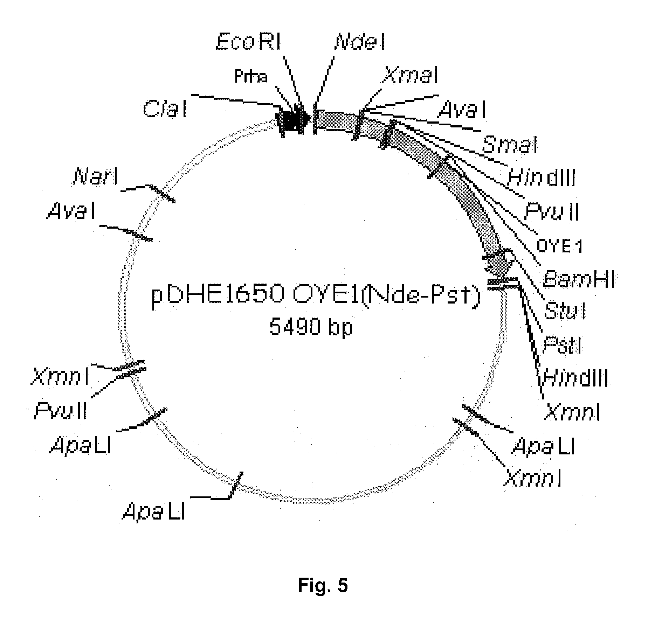

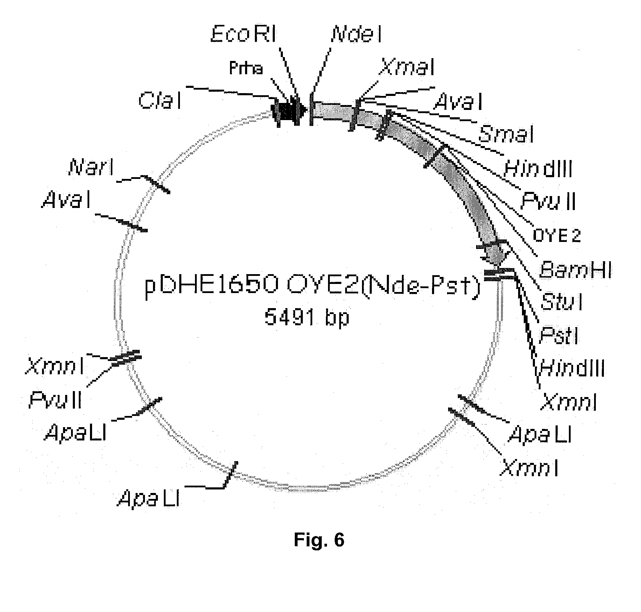

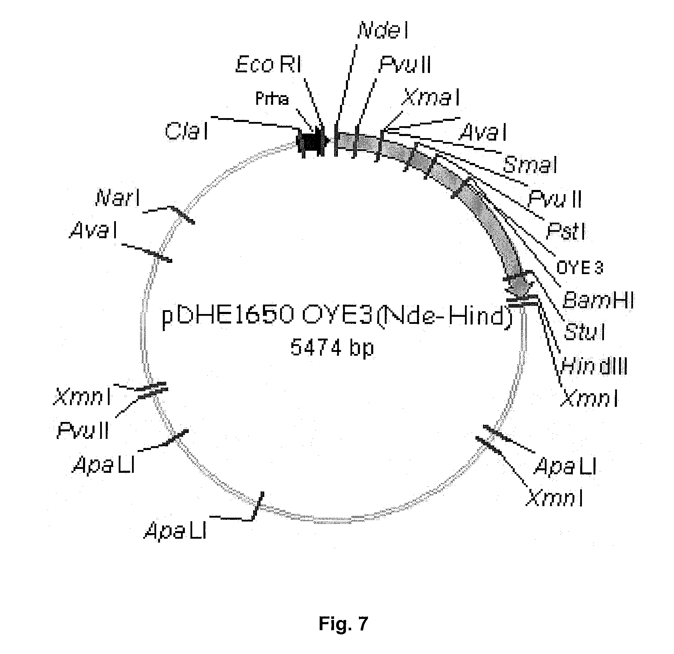

Folgende Plasmide wurden dabei erhalten:
pDHE1650 OYE1 (vgl. Figur 5)
pDHE1650 OYE2 (vgl. Figur 6)
pDHE1650 OYE3 (vgl. Figur 7)

### Beispiel 2: Biotransformationsexperimente

### a) Enzymproduktion

Die rekombinanten Stämme wurden in Luria-Broth-Medium (10 g/l Trypton, 10 g/l NaCl und 5 g/l Hefeextrakt), enthaltend 100 µg/ml Ampicillin, 100 µg/ml Streptomycin und 20 µg/ml Chloramphenico, gezüchtet. Die Proteinüberexpression wurde mit 0,1 mM IPTG für die beiden Chaperone und 0,5 g/l L-Rhamnose für OYE induziert. Die Kulturen wurden mit 120 Upm 22 Stunden bei 37°C geschüttelt. Die geernteten Zellen wurden bei - 20°C aufgewahrt.

### a) Biotransformation

Die Biotransformationen wurden in einem Reaktionsvolumen von 1 ml unter magnetischem Rühren bei 30°C über einen Zeitraum von 1 Stunde ausgeführt. Die Anfangskonzentrationen in dem Reaktionsgemisch waren folgende: 10 g Zelltrockenmasse/l, 10 % Isopropanol [Volumen/Volumen], 20 mM 4-Phenyl-3-butin-2-on, 5 mM EDTA, 2 mM NADP⁺, 1 U/ml Glucosedehydrogenase aus Thermoplasma acidophilum, 50 mM D-Glucose, 50 mM MES-Puffer, eingestellt mit KOH auf pH 6,8. Für Biotransformationen mit NADH wurde das NADPH-Regenerationssystem (NADP⁺, Glucose deyhdrogenase und Glucose) durch 15 mM NADH ersetzt.

### b) Auswertung der Versuche

Die Reaktionsgemische wurden mit Chloroform extrahiert und die Extrakte wurden durch Kapillargaschromatographie (GC) unter Verwendung eines Varian Star 3400 GC mit einer Supelco BPX5-Kapillarsäure (25 m x 0,32 mm Innendurchmesser mit einer Filmdicke der stationären Phase von 0,5 µm) und FID-Detektion nachgewiesen. Die Identität der Produkte wurde durch Co-Elution von Probenstandards und durch GC/MS-Daten belegt. Die GC/MS wurde auf einem Hewlett-Packard 5890, Serie II, Gaschromatographen, verbunden mit einem Hewlett-Packard 5972 TID Massenspektrometer unter Verwendung einer Restek RTX-5MS Kapillarsäule (30 m x 0,25 mm Innendurchmesser mit einer Filmdicke der stationären Phase von 0,25 µm) durchgeführt. Chromatogramme und die m/z-Verhältnisse wurden mit Hilfe der MSD ChemStation Software von Agilent Technologies analysiert. Zusätzlich wurden Datenbanken mit den Fragmentierungsmustern mit Hilfe der Wiley6 Library zur Identifizierung unbekannter Verbindungen durchsucht. Ein Brukman 300 MHZ GYRO NMR wurde zur Bestimmung der cis- und trans-Konfiguration des gebildeten Produktes 4-phenyl-3-butene-2-one verwendet. 1 D-WINNMR-Software wurde zur Datenanalyse verwendet und die erhaltenen Spektren wurden mit einem Standard von trans-4-Phenyl-3-buten-2-on mit einer Reinheit von 99 % verglichen.

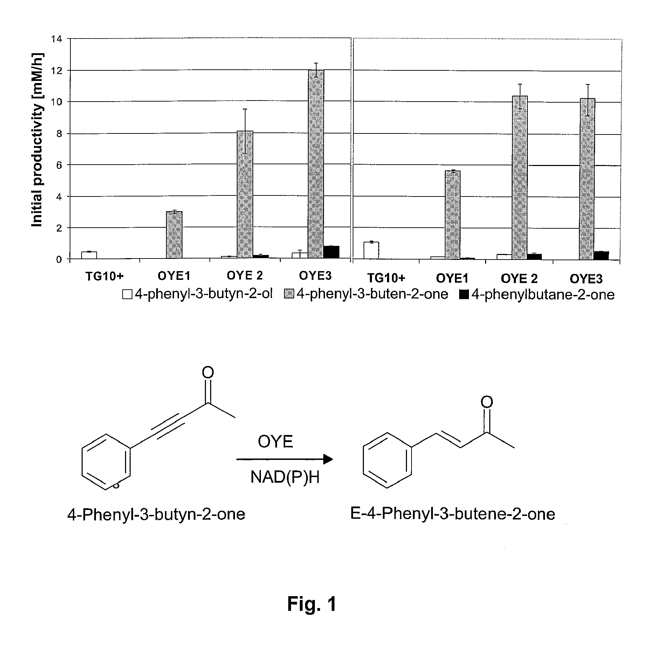

Die ermittelten Versuchsergebnisse, ausgedrückt über die anfängliche Produktivität (mM/h) sind in beiliegender Figur 1 zusammengefasst. Man beobachtet eine überraschend spezifische Bildung von E-4-Phenyl-3-buten-2-on.

## Patentansprüche

1. Verfahren zur enzymatischen Herstellung von Alkenon-Derivaten der allgemeinen Formel (2) aus α,β- ungesättigten Alkinon-Derivaten der allgemeinen Formel
(1) 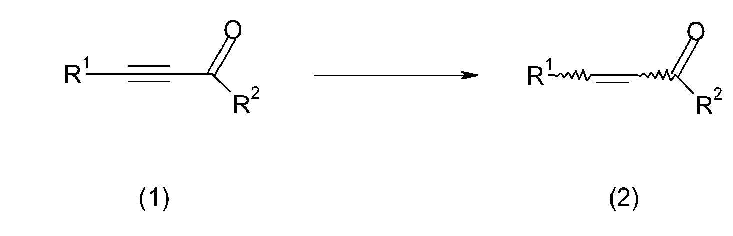
worin
R¹ für H, C₁-C₆- Alkyl, C₂-C₆- Alkenyl, oder einen gegebenenfalls substituierten carbo- oder heterocyclischen, aromatischen oder nichtaromatischen Rest steht, R² für H, C₁-C₆- Alkyl oder C₂-C₆-Alkenyl steht
durch Reduktion einer Verbindung der Formel (1) in Gegenwart einer Reduktase
(i) umfassend wenigstens eine der Polypeptidsequenzen SEQ ID NO:1, 2, 3, 5, 7 oder 9 oder
(ii) mit einer funktional äquivalenten Polypeptidsequenz, die mindestens 80% Sequenzidentität mit SEQ ID NO:1, 2, 3, 5, 7 oder 9 aufweist,
erfolgt.

2. Verfahren nach Anspruch 1, **dadurch gekennzeichnet, dass** die Reduktion ATPunabhängig mit NADPH oder NADH als Cofaktor durchgeführt wird.

3. Verfahren nach Anspruch 2, **dadurch gekennzeichnet, dass** der verwendete Cofaktor enzymatisch regeneriert wird.

4. Verfahren nach Anspruch 3, **dadurch gekennzeichnet, dass** die Cofaktor-Regenerierung durch Glucose-Dehydrogenase erfolgt.

5. Verfahren nach einem der vorhergehenden Ansprüche, **dadurch gekennzeichnet, dass** die Reduktion in einem wässrigen, wässrig-alkoholischen oder alkoholischen Reaktionsmedium erfolgt.

6. Verfahren nach einem der vorhergehenden Ansprüche, **dadurch gekennzeichnet, dass** die Reduktase immobilisiert vorliegt.

7. Verfahren nach einem der vorhergehenden Ansprüche, wobei das Enzym ausgewählt ist unter Reduktasen aus *Saccharomyces carlsbergensis* (Genbank Q02899), *Saccharomyces cerevisiae* (Genbank Q03558) und *Saccharomyces cerevisiae* (Genbank P41816).

8. Verfahren nach einem der vorhergehenden Ansprüche, wobei eine Verbindung der Formel (1) umgesetzt wird, worin
R¹ für gegebenenfalls substituiertes Aryl steht und R² für C₁-C₆- Alkyl steht.

9. Verfahren nach einem der vorhergehenden Ansprüche, wobei das E-Isomer von Verbindungen der Formel (2) anfällt.

10. Verfahren nach einem der vorhergehenden Ansprüche, wobei die Reaktion bei einer Temperatur im Bereich von 0 bis 45°C und/oder einem pH-Wert im Bereich von 6 bis 8 erfolgt.

11. Verwendung einer Verbindung der Formel (2), hergestellt nach einem Verfahren nach einem der vorhergehenden Ansprüche als Zwischenprodukt für die chemische oder enzymatische Wirkstoffsynthese.
